# EUROPEAN PATENT APPLICATION

(11) **EP 2 952 134 A1**
(43) Date of publication of application: **09.12.2015**
(21) Application number: 13873335.7
(22) Date of filing: 30.01.2013
(51) Int. Cl.: A61B 5/00, H04L 29/08

(54) **MONITOR REMOTE CONTROL METHOD AND SYSTEM THEREOF USING MOBILE TERMINAL**

(30) Priority: 29.01.2013 CN 201310033840
(71) Applicant: Edan Instruments, Inc., Shenzhen, Guangdong 518067 (CN)
(72) Inventor: SUN, Shuo, Shenzhen Guangdong 518067 (CN); ZHANG, Lei, Shenzhen Guangdong 518067 (CN)
(74) Representative: Brown, David Leslie
(86) International application number: PCT/CN2013/071132
(87) International publication number: WO 2014/117336

(57) **Abstract**

A monitor remote control method and system thereof using a mobile terminal. The system comprises: a mobile terminal, a monitor terminal and a network terminal. The mobile terminal comprises an instruction setting unit for inputting setting instructions and sending the setting instruction to the network terminal. The network terminal comprises a data and instruction receiving unit for receiving the setting instructions, and a wireless connection unit for building a wireless connection between the mobile terminal and the monitor terminal. The monitor terminal comprises a setting execution unit for receiving and executing the setting instructions sent by a data and instruction sending unit of the network terminal. The system enables medical staffs when away from bedside to know information and parameter conditions of patients in real time and to set the settings of the monitor.

## Description

### TECHNICAL FIELD

The invention relates to a monitor remote control method and system thereof, particularly to a monitor remote control method and system thereof using a mobile terminal.

### BACKGROUND OF THE INVENTION

ECG, body temperature, blood pressure and other physiological parameters of the human body and its changes are common parameters used by doctors to diagnose the disease. In order to determine the aforementioned body parameters, clinical monitors came into being. Early clinical monitors used a stand-alone machine, that is to say, doctors needed to monitor a variety of physiological parameters of the patient around him in real-time. However, this not only increased the workload of doctors but also reduced the efficiency of doctors when the patient is under the stable conditions, in the case that the patient's condition suddenly deteriorated, it may also cause that the doctor cannot know sign parameter information in the first time, which delays the valuable treatment time of the patient and may even lead to worse consequences.

Telemonitoring has been an important development direction in a telemedicine research field in recent years, and integrated electronic information technology, communications technology, computer technology and medical engineering as a whole. Remote monitoring is a method in which the physiological functions of a distant object are studied through continuous monitoring for physiological parameters, and the object can receive the treatment from the doctors and specialists in a timely fashion when he has abnormal physiological functions. With the improvement of people's living standards, people begin to more and more care high quality medical service, and hope to still receive the monitoring and treatment when they are at home and travelling.

The rapid development of wireless communication technology and the increasing levels of software design currently enable wireless communication between a monitor and a mobile terminal. For example, Patent No. 200710003350.3 with the title of "A Remote Wireless ECG Monitor in Real Time" has solved problem of monitoring patient information signs in real time in a mobile terminal with GSM / GPRS or CDMA wireless digital communication technology. Currently, between the monitor and the mobile terminal, the care information of the patient only can be sent to the mobile terminal, when medical staffs are not in the patient's bedside but would like to know the real-time status of the patient, the patient's information on the monitor can be transmitted to the mobile terminal by wireless means, and the medical staffs can browse patient information parameters through the mobile terminal, however, if the monitor need be operated, for example, when the information such as the set parameters of the monitor obviously does not match with the cared patients or the patient information (including: name, gender, responsibility doctor, blood type, etc.) need be input into the monitor, the health care need directly set the monitor in the patient's bedside, which is very inconvenient.

### SUMMARY OF THE INVENTION

### THE TECHNICAL PROBLEM

For the defect of the abovementioned prior art, the invention provides a monitor remote control method and system thereof using a mobile terminal.

### THE TECHNICAL SOLUTION

A monitor remote control system using a mobile terminal includes a mobile terminal, a network terminal and a monitor terminal which are connected sequentially;
the mobile terminal includes:
a instruction importing unit for inputting setting instructions for setting control parameters of the monitor terminal, the control parameters being the parameters for controlling the function of each monitor module of the monitor;
an instruction sending unit connected with the instruction importing unit to receive the setting instructions, add the setting instructions to communication instructions and send the communication instructions to the network terminal;
a data receiving unit connected with the network terminal to receive the communication instructions sent by the network terminal;
a data display unit connected with the data receiving unit to receive the communication instructions sent by the data receiving unit as well as extract and display the monitoring data in the communication instructions;
the network terminal includes:
   a wireless connection unit for building a wireless connection between the mobile terminal and the monitor terminal respectively;
   a data and instruction receiving unit for connecting the instruction sending unit in the mobile terminal and a monitoring data sending unit in the monitor terminal to receive the communication instructions;
   a data and instruction sending unit for receiving the communication instructions sent by the data and instruction receiving unit and sending the communication instructions to the data receiving unit of the mobile terminal and the monitor terminal, respectively;
the monitor terminal includes:
   an instruction receiving unit for connecting the data and instruction sending unit in the network terminal to receive the communication instructions sent by the data and instruction sending unit;
   an instruction execution unit for connecting the instruction receiving unit to extract the setting instructions from the received communication instructions and carry out corresponding operations based on the setting instructions;
   a monitoring data collecting unit for connecting the monitoring data sending unit to collect and send the monitoring data;
   a monitoring data sending unit for connecting the data and instruction receiving unit of the network terminal to add the monitoring data to the communication instructions and send the communication instructions to the network terminal.

Furthermore, the mobile terminal still includes:
an identity and authority information importing unit for imputing identity and authority verification information;
a request unit for identity and authority verification for connecting the identity and authority information importing unit and the network terminal to send the identity and authority information to the network terminal.

Furthermore, the network terminal still includes:
An identity and authority verification unit for connecting the request unit for the identity and authority verification to receive the identity and authority verification information sent by the request unit for the identity and authority verification in the mobile terminal and judge which use authority comprising browse authority and control authority the user possesses.

Furthermore, a modulation unit is still provided between the data and instruction receiving unit and the data and instruction sending unit in the network terminal, the modulation unit is used for modulating the received setting instructions or the monitoring data into the data information which can be identified by the monitor terminal or the mobile terminal, and sending them to the data and instruction sending unit again.

Furthermore, the input and verified identity and authority information includes input verification instruction or input username and password.

Furthermore, the terminal still includes an error handling unit, the error handling unit is connected with the identity and authority verification unit and the instruction receiving unit in the monitor terminal to terminate the operation and send error prompt information to the mobile terminal if it does not possess the use authority when the identify and authority information is verified, or when control parameters of the monitor terminal fail to be set.

A monitor remote control method using a mobile terminal includes the following steps:
a monitor terminal, a network terminal and a mobile terminal are wirelessly interlinked;
the monitor terminal receives communication instructions comprising browse instructions and sent by the mobile terminal, sends collected monitoring data based on the browse instructions to the network terminal, and sends the monitoring data processed by the network terminal to the mobile terminal for displaying the monitoring data;
the mobile terminal adds setting information in the communication instructions and sends setting request to the network terminal based on the setting information;
the network terminal processes the setting request and sends setting instructions to the monitor terminal;
the monitor terminal receives the setting instructions and carries out the setting instructions.

Furthermore, after the monitor terminal, the network terminal and the mobile terminal are wirelessly interlinked, the method still includes:
identity verification information is input into the mobile terminal and sent to the network terminal, based on the identity verification information, the network terminal judge whether the user possesses use authority, if the user possesses the authority, he judges which use authority he possesses, authority information includes browse authority and control authority, if the user possesses the browse authority, the mobile terminal is allowed to add the browse instructions to the communication instructions and send the browse instructions to the monitor terminal via the network terminal, if the user possesses the control authority, the mobile terminal is allowed to add the browse instructions and the control instructions to the communication instructions and send the browse instructions the control instructions to the monitor terminal via the network terminal.

Furthermore, after the monitor terminal receives the setting instructions and carries out the setting instructions, the method still includes:
the setting is judged whether it is successful, if the data displayed by the mobile terminal are not the set monitoring data, it is judged that the data setting fails, the operation is terminated and error prompt information is sent to the mobile terminal; if the data displayed by the mobile terminal are the set monitoring data, it is judged that the data is successfully set, and the mobile terminal displays the monitoring data which have been successfully set.

Furthermore, after it is judged which use authority the user possesses, the method still includes:
it is judged whether the user possesses the use authority, if he does not possess the authority, the operation is terminated and the error prompt information is sent to the mobile terminal.

### EFFECTIVE RESULTS

The invention solves the problem that: when medical staffs are not in the patient's bedside, they still can know the information and parameters of the patient in real time and set the parameters of the monitor, which is very convenient.

### BRIEF DESCRIPTION OF THE DRAWINGS

In order to be described easily, the invention is described with the following preferred examples and figures in details.
Figure 1 is a structural drawing of a monitor remote control system using a mobile terminal;
Figure 2 is a flow drawing of a monitor remote control method using a mobile terminal;
Figure 3 is a concrete flow drawing of a monitor remote control method using a mobile terminal.

### DETAILED DESCRIPTION OF THE EMBODIMENT

The invention will be further described in more details and the purposes, the technical solution and the advantages of the invention will be more apparent with the combination of the following drawings and embodiments. It shall be understood that the embodiments described herein are only used for explaining the invention but do not limit the invention.

The invention is further described in more details with the combination with embodiments and drawings.

The invention aims at overcoming the defects of the prior art and provides a monitor remote control method and system thereof using mobile terminal, wherein a mobile terminal and wireless technology are used to control a monitor to meet the requirements of clinical medical staffs. The mobile terminal includes but is not limited to a mobile phone and a tablet computer. The wireless communication technology includes but is not limited to Bluetooth, WIFI and infra-red.

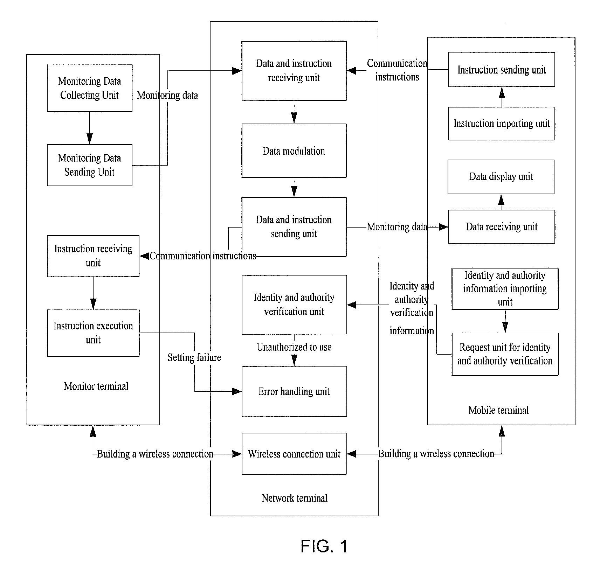

To achieve this object, the invention adopts the following technical solution:
As shown in FIG. 1, a monitor remote control system using a mobile terminal is provided and comprises a mobile terminal, a network terminal and a monitor terminal; the monitor terminal includes a monitor and other instrument;

The mobile terminal includes:
a instruction importing unit for inputting setting instructions for setting control parameters of the monitor terminal, the control parameters being the parameters for controlling the function of each monitor module of the monitor; an instruction sending unit connected with the instruction importing unit to receive the setting instructions, add the setting instructions to communication instructions and send the communication instructions to the network terminal;
a data receiving unit connected with the network terminal to receive the communication instructions sent by the network terminal; a data display unit connected with the data receiving unit to receive the communication instructions sent by the data receiving unit as well as extract and display the monitoring data in the communication instructions; an identity and authority information importing unit for imputing identity and authority verification information;
a request unit for identity and authority verification for connecting the identity and authority information importing unit and the network terminal to send the identity and authority information to the network terminal.

The network terminal includes:
a wireless connection unit for building a wireless connection between the mobile terminal and the monitor terminal respectively;
a data and instruction receiving unit for connecting the instruction sending unit in the mobile terminal and a monitoring data sending unit in the monitor terminal to receive the communication instructions;
a data and instruction sending unit for receiving the communication instructions sent by the data and instruction receiving unit and sending the communication instructions to the data receiving unit of the mobile terminal and the monitor terminal, respectively;
an identity and authority verification unit for connecting the request unit for the identity and authority verification to receive the identity and authority verification information sent by the request unit for the identity and authority verification in the mobile terminal and judge which use authority comprising browse authority and control authority the user possesses.
a modulation unit which is still provided between the data and instruction receiving unit and the data and instruction sending unit in the network terminal, and used for modulating the received setting instructions or the monitoring data into the data information which can be identified by the monitor terminal or the mobile terminal, and sending them to the data and instruction sending unit again.
an error handling unit which is connected with the identity and authority verification unit and the instruction receiving unit in the monitor terminal to terminate the operation and
send error prompt information to the mobile terminal if it does not possess the use authority when the identify and authority information is verified, or when control parameters of the monitor terminal fail to be set.
the monitor terminal includes:
   an instruction receiving unit for connecting the data and instruction sending unit in the network terminal to receive the communication instructions sent by the data and instruction sending unit;
   an instruction execution unit for connecting the instruction receiving unit to extract the setting instructions from the received communication instructions and carry out corresponding operations based on the setting instructions;
   a monitoring data collecting unit for connecting the monitoring data sending unit to collect and send the monitoring data;
   a monitoring data sending unit for connecting the data and instruction receiving unit of the network terminal to add the monitoring data to the communication instructions and send the communication instructions to the network terminal.

The system can preset a number of fixed users and casual users, the fixed users have stable identity information and use authority, such as the attending physician, the system will assign a common fixed ID to the fixed users while the casual users do not have stable identity information and use authority, that is to say, the casual user may be changed at any time, such as a nurse or patient's family, the system will assign a casual ID with prescription or specified number of times to the casual user. The next operation can be carried out after setting is successful.

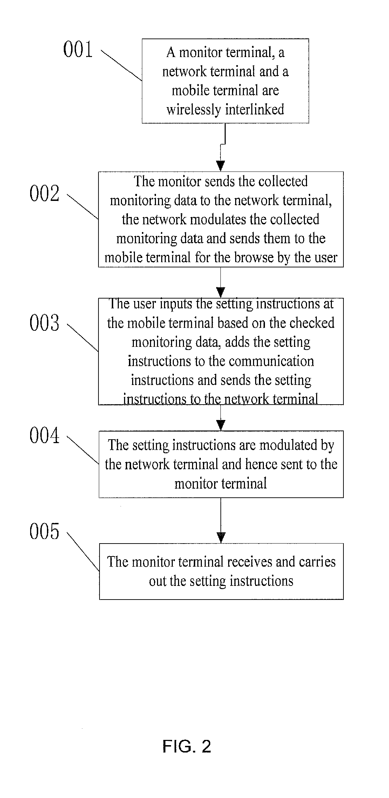

As shown in FIG. 2, A monitor remote control method using a mobile terminal is provided and includes the following steps:
001. a monitor terminal, a network terminal and a mobile terminal are wirelessly interlinked;
002. The monitor terminal receives communication instructions comprising browse instructions and sent by the mobile terminal, sends collected monitoring data based on the browse instructions to the network terminal, and sends the monitoring data processed by the network terminal to the mobile terminal for displaying the monitoring data;
003. the mobile terminal adds setting information in the communication instructions and sends setting request to the network terminal based on the setting information;
004. the network terminal processes the setting request and sends setting instructions to the monitor terminal;
005. the monitor terminal receives the setting instructions and carries out the setting instructions.

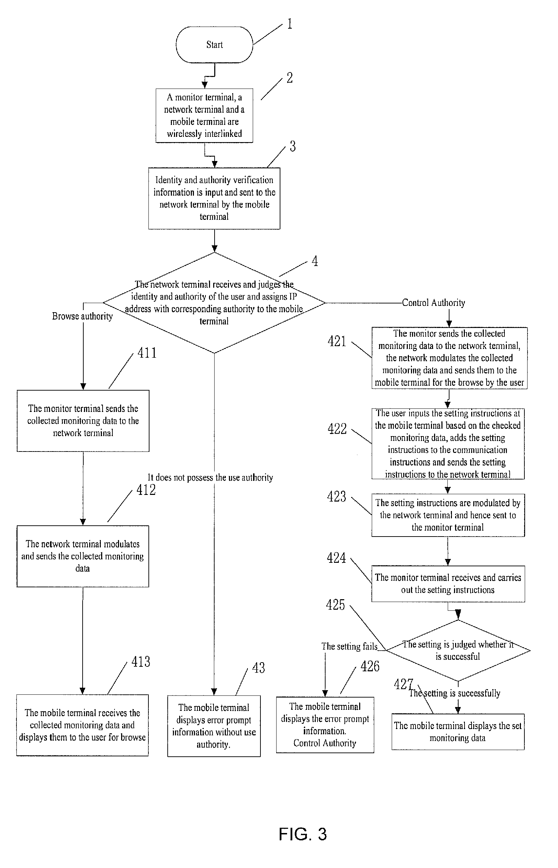

As shown in Figure 3, a monitor remote control method using a mobile terminal is provided and includes the following steps:
1. A monitor, a mobile terminal and a network terminal are turned on.
2. The mobile terminal, the network terminal and the monitor terminal are interlinked, the wireless communication interconnection of a peer-to-peer device is established among the mobile terminal, the network terminal and the monitor terminal, wireless communication manners such as Bluetooth / WIFI are adopted.
3. Identity and authority verification information which can be verification instructions or a user name and passwords is input, the mobile terminal sends the identity authority verification information to the network terminal.
4. The network terminal sends the identity authority verification information based on the mobile terminal, and judges which use authority it possesses, the use authority includes browse authority and control authority;
   411. If it possesses the browse authority, the mobile terminal is allowed to add the browse instructions to the communication instructions and sends the brows instructions to the monitor terminal via the network terminal, after receiving the instructions, the monitor terminal adds the collected patient's care data to the communication instructions and sends the collected patient's care data to the network terminal;
   412. The network terminal receives the monitoring data sent by the monitor terminal and sends the modulated monitoring data to the mobile terminal of the user,
   413. The mobile terminal receives the modulated monitoring data, extracts the monitoring data in the communication instructions and displays them to the user for browse, the data sent by the monitor terminal include history data and real-time monitoring data of the patient.
   421. If it possesses the control authority, the mobile terminal is allowed to add the browse instructions and the control instructions to the communication instructions and sends the browse instructions and the control instructions to the monitor terminal via the network terminal, after receiving the instructions, the monitor terminal sends the collected patient' monitoring data to the network terminal, the collected patient' monitoring data are modulated via the network terminal and then sent to the mobile terminal of the user for browse.
   422. The user inputs the setting instructions at the mobile terminal based on the checked monitoring data, adds the setting instructions to the communication instructions and sends the setting instructions to the network terminal.
   423. The network terminal receives the communication instructions sent by the mobile terminal, and sends the communication instructions to the monitor terminal after the communication instructions are modulated by the network terminal.
   424. The monitor terminal receives the communication instructions sent by the network terminal, extracts the setting instruction from the communication instructions, and executes the corresponding operation based on the setting instructions.
   425. It is judged whether the monitor terminal is disposed successfully. If the data displayed by the mobile terminal is the set monitoring data, it is judged that the data are set successfully; if the data displayed by the mobile terminal is not the set monitoring data, it is judged that the data are set unsuccessfully.
   426. The mobile terminal displays error prompt information.
      When the control parameters of the monitor terminal are set unsuccessfully, the network terminal sends the error prompt information to the mobile terminal for display to inform the user.
   427. The monitoring data which are set successfully are displayed.
      When the control parameters of the monitor terminal are set successfully, the mobile terminal displays the monitoring data which are set successfully and browsed by the user.
   43. If it does not possess the use authority, the network terminal processes the error prompt information and sends it to the mobile terminal for display to inform the user. The above-mentioned are only preferred embodiments of the invention and do not limit the invention, Any modification, equal replacement and improvement made within the spirit and the principle of the invention shall be included in the protection scope of the invention.

## Claims

1. A monitor remote control system using a mobile terminal, the system comprising the mobile terminal, a network terminal and a monitor terminal which are connected sequentially, wherein:
the mobile terminal comprises:
a instruction importing unit for inputting setting instructions for setting control parameters of the monitor terminal, the control parameters being the parameters for controlling the function of each monitor module of the monitor;
an instruction sending unit connected with the instruction importing unit to receive the setting instructions, add the setting instructions to communication instructions and send the communication instructions to the network terminal;
a data receiving unit connected with the network terminal to receive the communication instructions sent by the network terminal;
a data display unit connected with the data receiving unit to receive the communication instructions sent by the data receiving unit as well as extract and display the monitoring data in the communication instructions;
the network terminal comprises:
a wireless connection unit for building a wireless connection between the mobile terminal and the monitor terminal respectively;
a data and instruction receiving unit for connecting the instruction sending unit in the mobile terminal and a monitoring data sending unit in the monitor terminal to receive the communication instructions;
a data and instruction sending unit for receiving the communication instructions sent by the data and instruction receiving unit and sending the communication instructions to the data receiving unit of the mobile terminal and the monitor terminal, respectively;
the monitor terminal comprises:
an instruction receiving unit for connecting the data and instruction sending unit in the network terminal to receive the communication instructions sent by the data and instruction sending unit;
an instruction execution unit for connecting the instruction receiving unit to extract the setting instructions from the received communication instructions and carry out corresponding operations based on the setting instructions;
a monitoring data collecting unit for connecting the monitoring data sending unit to collect and send the monitoring data;
a monitoring data sending unit for connecting the data and instruction receiving unit of the network terminal to add the monitoring data to the communication instructions and send the communication instructions to the network terminal.

2. A monitor remote control system using a mobile terminal according to claim 1, wherein, the mobile terminal still comprises:
an identity and authority information importing unit for imputing identity and authority verification information;
a request unit for identity and authority verification for connecting the identity and authority information importing unit and the network terminal to send the identity and authority information to the network terminal.

3. A monitor remote control system using a mobile terminal according to claim 2, wherein, the network terminal still comprises:
an identity and authority verification unit for connecting the request unit for the identity and authority verification to receive the identity and authority verification information sent by the request unit for the identity and authority verification in the mobile terminal and judge which use authority comprising browse authority and control authority the user possesses.

4. A monitor remote control system using a mobile terminal according to claim 3, wherein, a modulation unit is still provided between the data and instruction receiving unit and the data and instruction sending unit in the network terminal, the modulation unit is used for modulating the received setting instructions or the monitoring data into the data information which can be identified by the monitor terminal or the mobile terminal, and sending them to the data and instruction sending unit again.

5. A monitor remote control system using a mobile terminal according to claim 4, wherein, the input and verified identity and authority information comprises input verification instruction or input username and password.

6. A monitor remote control system using a mobile terminal according to claim 5, wherein, the network terminal still comprises an error handling unit, the error handling unit is connected with the identity and authority verification unit and the instruction receiving unit in the monitor terminal to terminate the operation and send error prompt information to the mobile terminal if it does not possess the use authority when the identify and authority information is verified, or when control parameters of the monitor terminal fail to be set.

7. A monitor remote control method using a mobile terminal for realizing claim 1, wherein the method comprises the following steps:
a monitor terminal, a network terminal and a mobile terminal are wirelessly interlinked;
the monitor terminal receives communication instructions comprising browse instructions and sent by the mobile terminal, sends collected monitoring data based on the browse instructions to the network terminal, and sends the monitoring data processed by the network terminal to the mobile terminal for displaying the monitoring data;
the mobile terminal adds setting information in the communication instructions and sends setting request to the network terminal based on the setting information;
the network terminal processes the setting request and sends setting instructions to the monitor terminal;
the monitor terminal receives the setting instructions and carries out the setting instructions.

8. A monitor remote control method using a mobile terminal according to claim 7, wherein, after the monitor terminal, the network terminal and the mobile terminal are wirelessly interlinked, the method still comprises:
identity verification information is input into the mobile terminal and sent to the network terminal, based on the identity verification information, the network terminal judge whether the user possesses use authority, if the user possesses the authority, he judges which use authority he possesses, authority information comprises browse authority and control authority, if the user possesses the browse authority, the mobile terminal is allowed to add the browse instructions to the communication instructions and send the browse instructions to the monitor terminal via the network terminal, if the user possesses the control authority, the mobile terminal is allowed to add the browse instructions and the control instructions to the communication instructions and send the browse instructions the control instructions to the monitor terminal via the network terminal.

9. A monitor remote control method using a mobile terminal according to claim 8, wherein, after the monitor terminal receives the setting instructions and carries out the setting instructions, the method still comprises:
the setting is judged whether it is successful, if the data displayed by the mobile terminal are not the set monitoring data, it is judged that the data setting fails, the operation is terminated and error prompt information is sent to the mobile terminal; if the data displayed by the mobile terminal are the set monitoring data, it is judged that the data is successfully set, and the mobile terminal displays the monitoring data which have been successfully set.

10. A monitor remote control method using a mobile terminal according to claim 9, wherein, after it is judged which use authority the user possesses, the method still comprises:
it is judged whether the user possesses the use authority, if he does not possess the authority, the operation is terminated and the error prompt information is sent to the mobile terminal.
